(19) Europäisches Patentamt
European Patent Office
Office européen des brevets

(11) Veröffentlichungsnummer: **0 247 486**
**A1**

(12) # EUROPÄISCHE PATENTANMELDUNG

(21) Anmeldenummer: **87107166.8**

(22) Anmeldetag: **18.05.87**

(51) Int. Cl.[4]: **C 07 D 493/08**, C 07 D 493/18,
A 01 N 43/90
//
(C07D493/08, 307:00, 307:00),
(C07D493/18, 317:00, 307:00,
307:00)

(30) Priorität: **27.05.86 DE 3617715**

(43) Veröffentlichungstag der Anmeldung: **02.12.87**
**Patentblatt 87/49**

(84) Benannte Vertragsstaaten: **BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Knops, Hans-Joachim, Dr., Köpenicker Strasse 35, D-4019 Monheim (DE)**
Erfinder: **Steinbeck, Karl, Dr., c/o Mobay Corporation P.O. Box 4913 Hawthorn Road, Kansas City Missouri 64120 (US)**
Erfinder: **Babczinski, Peter, Dr., In der Lohrenbeck 11, D-5600 Wuppertal 1 (DE)**
Erfinder: **Santel, Hans-Joachim. Dr., Grünstrasse 9a, D-5090 Leverkusen 1 (DE)**
Erfinder: **Schmidt, Robert R., Dr., Im Waldwinkel 110, D-5060 Bergisch Gladbach 2 (DE)**

(54) **Oxa-bicyclo [2.2.1] hepten- Derivate.**

(57) Die Erfindung betrifft neue Oxa-bicyclo[2,2,1]hepten-Derivate der Formel

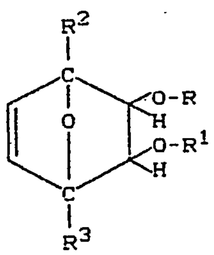

(I)

in welcher

R für Wasserstoff oder gegebenenfalls substituiertes Aralkyl steht,

$R^1$ für Wasserstoff, Alkyl, gegebenenfalls substituiertes Aralkyl oder für den Rest $-CO-R^4$ steht, worin

$R^4$ für Alkyl oder gegebenenfalls substituiertes Phenyl oder für den Rest $-NH-R^5$ steht, worin

$R^5$ für Alkyl oder gegebenenfalls substituiertes Phenyl steht und

$R^2$ und $R^3$ gleich oder verschieden sind und für Alkyl stehen, mehrere Verfahren zu deren Herstellung und ihre Verwendung als Herbizide sowie neue Zwischenprodukte.

EP 0 247 486 A1

BAYER AKTIENGESELLSCHAFT 5090 Leverkusen, Bayerwerk
Konzernverwaltung RP
Patentabteilung KM/ABc
Ib

# Oxa-bicyclo[2.2.1]hepten-Derivate

Die Erfindung betrifft neue Oxa-bicyclo[2.2.1]hepten-Derivate, mehrere Verfahren zu ihrer Herstellung und ihre Verwendung als Herbizide sowie neue Zwischenprodukte zu deren Herstellung.

Es ist bereits bekannt, daß zahlreiche Oxa-bicyclo[2.2.1]-heptan-Derivate, herbizide Eigenschaften besitzen. So kann zum Beispiel exo-1-Methyl-4-(1-methylethyl)-2-(2-methylphenylmethoxy)-7-oxabicyclo[2.2.1]heptan zur Bekämpfung von Unkräutern eingesetzt werden (vgl. EP-A-81 893). Diese Stoffe zeigen jedoch in einigen Kulturpflanzen keine zufriedenstellende Selektivität.

Es wurden nun neue Oxa-bicyclo[2.2.1]hepten-Derivate der Formel (I)

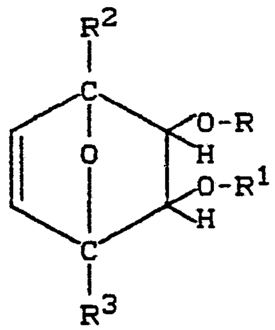

(I)

in welcher

R für Wasserstoff oder gegebenenfalls substituiertes Aralkyl steht,

$R^1$ für Wasserstoff, Alkyl, Alkenyl, Alkinyl, gegebenenfalls substituiertes Aralkyl oder für den Rest $-CO-R^4$ steht,
worin

$R^4$ für Alkyl oder gegebenenfalls substituiertes Phenyl oder für den Rest $-NH-R^5$ steht,
worin

$R^5$ für Alkyl oder gegebenenfalls substituiertes Phenyl steht und

$R^2$ und $R^3$ gleich oder verschieden sind und für Alkyl stehen,

gefunden.

Die neuen Oxa-bicyclo[2.2.1]hepten-Derivate der allgemeinen Formel (I) können in der exo- oder endo-Form bzw. als Gemische dieser beiden Formen vorliegen und sind sowohl in der exo- als auch in der endo-Form und als Gemisch Gegenstand der vorliegenden Erfindung.

Weiterhin wurde gefunden, daß man die neuen Oxa-bicyclo-[2.2.1]hepten-Derivate der Formel (I) erhält, wenn man

A) Diolcarbonate der Formel (II)

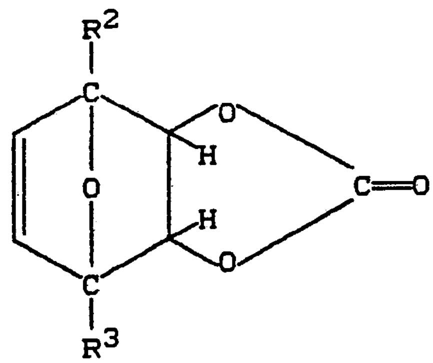

(II)

in welcher

$R^2$ und $R^3$ gleich oder verschieden sind und für Alkyl stehen,

gegebenenfalls in Gegenwart eines Verdünnungsmittels und in Gegenwart einer Base hydrolisiert,

oder wenn man

B) die nach Verfahren (A) erhältlichen Diole der Formel (I-a)

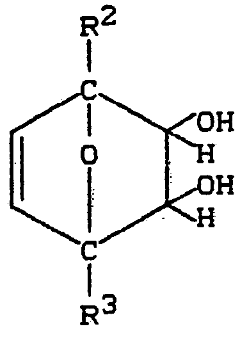

(I-a)

in welcher

$R^2$ und $R^3$ die oben angegebene Bedeutung haben,

mit Halogeniden der Formel (III)

$R^6$ - Hal (III)

in welcher

$R^6$ für gegebenenfalls substituiertes Aralkyl steht und

Hal für Halogen steht,

gegebenenfalls in Gegenwart eines Verdünnungsmittels und gegebenenfalls in Gegenwart eines Säurebindemittels umsetzt,

oder wenn man

C) Oxa-bicyclo[2.2.1]hepten-Derivate der Formel (I-b)

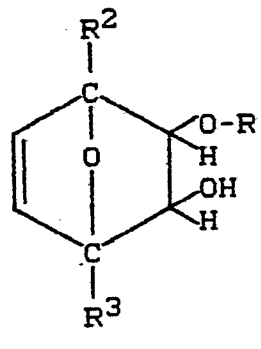

(I-b)

in welcher

$R^2$, $R^3$ und R die oben angegebene Bedeutung haben,

α) mit Halogeniden der Formel (IV)

$R^{1-1}$-Hal (IV)

in welcher

$R^{1-1}$ für Alkyl, Alkenyl, Alkinyl oder gegebenenfalls substituiertes Aralkyl steht und

Hal für Halogen steht,

gegebenenfalls in Gegenwart eines Verdünnungsmittels und gegebenenfalls in Gegenwart einer Base umsetzt, oder

β) mit Acylhalogeniden der Formel (V)

$$R^4-\underset{\underset{O}{\|}}{C}-Hal \qquad (V)$$

in welcher

$R^4$ die oben angegebene Bedeutung hat und

Hal für Halogen steht,

gegebenenfalls in Gegenwart eines Säurebindemittels sowie gegebenenfalls in Gegenwart eines Verdünnungsmittels umsetzt,

oder

γ) mit Isocyanaten der Formel (VI)

$R^5$-N=C=O (VI)

in welcher
$R^5$ die oben angegebene Bedeutung hat,

gegebenenfalls in Gegenwart eines Verdünnungsmittels umsetzt;

oder wenn man

D) Ether-Derivate der Formel (I-c)

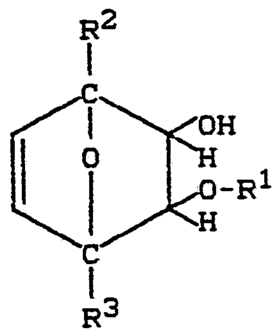

(I-c)

in welcher

$R^1$, $R^2$ und $R^3$ die oben angegebene Bedeutung haben,

mit Verbindungen der Formel (III)

$R^6$-Hal (III)

in welcher

$R^6$ die oben angegebene Bedeutung hat und

Hal für Halogen steht,

gegebenenfalls in Gegenwart einer Base und gegebenenfalls in Gegenwart eines Verdünnungsmittels umsetzt.

Schließlich wurde gefunden, daß sich die neuen Oxa-bicyclo[2.2.1]hepten-Derivate der Formel (I) durch eine sehr gute herbizide Wirksamkeit auszeichnen.

Überraschenderweise besitzen die erfindungsgemäßen Oxa-bicyclo[2.2.1]hepten-Derivate der Formel (I) wesentlich bessere herbizid-selektive Eigenschaften in wichtigen Kulturpflanzen, wie insbesondere Reis, als das bekannte exo-1-Methyl-4-(1-methylethyl)-2-(2-methylphenylmethoxy)-7-oxabicyclo[2.2.1]heptan, welches ein konstitutionell ähnlicher Wirkstoff gleicher Wirkungsart ist.

Die erfindungsgemäßen Oxa-bicyclo[2.2.1]hepten-Derivate sind durch die Formel (I) allgemein definiert. In dieser Formel stehen vorzugsweise

R für Wasserstoff oder gegebenenfalls einfach oder mehrfach, gleich oder verschieden substituiertes Aralkyl mit 6 bis 10 Kohlenstoffatomen im Arylteil und 1 bis 4 Kohlenstoffatomen im Alkylteil, wobei als Arylsubstituenten ausgewählt sind: Halogen, Alkyl, Alkoxy oder Alkylthio mit jeweils 1 bis 4 Kohlenstoffatomen, Halogenalkyl, Halogenalkoxy und Halogenalkylthio mit jeweils 1 bis 4 Kohlenstoffatomen und 1 bis 9 gleichen oder verschiedenen Halogenatomen, wie insbesondere Fluor- und Chloratomen,

$R^1$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, gegebenenfalls einfach oder mehrfach, gleich oder verschieden substituiertes Aralkyl mit 6 bis 10 Kohlenstoffatomen im Arylteil und 1 bis 4 Kohlenstoffatomen im Alkylteil steht, wobei als Arylsubstituenten die bei R genannten Arylsubstituenten infrage kommen; ferner für den Rest $-COR^4$ steht, wobei

$R^4$ für Alkyl mit 1 bis 4 Kohlenstoffatomen oder gegebenenfalls einfach oder mehrfach, gleich oder verschieden substituiertes Phenyl steht, wobei als Phenylsubstituenten die bei R genannten Arylsubstituenten infrage kommen, oder für den Rest $-NHR^5$ steht, wobei

$R^5$ für Alkyl mit 1 bis 4 Kohlenstoffatomen oder für gegebenenfalls einfach oder mehrfach, gleich oder verschieden substituiertes Phenyl steht, wobei als Phenylsubstituenten die bei R genannten Arylsubstituenten infrage kommen, und

$R^2$ und $R^3$ gleich oder verschieden sind und für Alkyl mit 1 bis 6 Kohlenstoffatomen stehen.

Besonders bevorzugt sind diejenigen Verbindungen der Formel (I), die in der exo-Form vorliegen und in denen

R für Wasserstoff oder für jeweils einfach bis dreifach, gleich oder verschieden durch Wasserstoff, Fluor,

Chlor, Methyl, Ethyl, iso-Propyl, n-Propyl, Trifluormethyl, Trifluormethoxy, Trifluormethylthio, Methoxy, Ethoxy, Methylthio und Ethylthio substituiertes Benzyl oder Phenethyl steht,

und

$R^1$ für Wasserstoff, Methyl, Ethyl, n-Propyl, iso-Propyl, Allyl oder Propinyl steht; ferner für Benzyl steht, welches gegebenenfalls einfach bis dreifach, gleich oder verschieden durch Fluor, Chlor, Brom, Iod, Methyl und Ethyl substituiert ist; sowie für den Rest $-COR^4$ steht, wobei

$R^4$ für Methyl, Ethyl, Phenyl oder den Rest $-NHR^5$ steht, wobei

$R^5$ für Methyl, Ethyl oder Phenyl steht und

$R^2$ und $R^3$ gleich oder verschieden sind und für Methyl oder Ethyl stehen.

Ganz besonders bevorzugt sind diejenigen Verbindungen der Formel (I), welche in der exo-Form vorliegen und in denen

R für Wasserstoff oder für ein in ortho-Stellung jeweils durch Wasserstoff, Fluor, Chlor, Methyl, Ethyl, iso-Propyl, n-Propyl, Trifluormethyl, Trifluormethoxy, Trifluormethylthio, Methoxy, Ethoxy, Methylthio oder Ethylthio substituiertes Benzyl steht und

$R^1$, $R^2$, $R^3$, $R^4$ und $R^5$ die oben als besonders bevorzugt angegebene Bedeutung haben.

Als Beispiele für Oxa-bicyclo[2.2.1]hepten-Derivate der Formel (I) seien die in der folgenden Tabelle formelmäßig aufgeführten Verbindungen genannt:

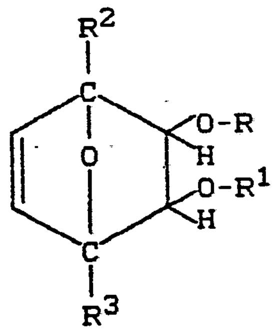

(I)

Tabelle 1

| $R^1$ | $R^2$ | $R^3$ | Ar |
|---|---|---|---|
| H | $CH_3$ | $CH_3$ | 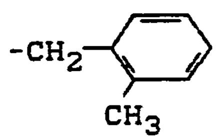 |
| H | $CH_3$ | $CH_3$ | 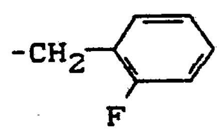 |
| H | $CH_3$ | $CH_3$ | 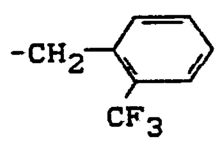 |
| H | $CH_3$ | $CH_3$ | 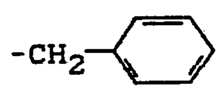 |
| $C_2H_5$ | $CH_3$ | $CH_3$ | H |
| $n-C_3H_7$ | $CH_3$ | $CH_3$ | H |

Verwendet man exo-cis-1,4-Dimethyl-7-oxabicyclo[2.2.1]-hept-5-en-2,3-diolcarbonat und Natronlauge als Ausgangssubstanzen, so kann der Verlauf des erfindungsgemäßen Verfahrens (A) durch das folgende Formelschema wiedergegeben werden:

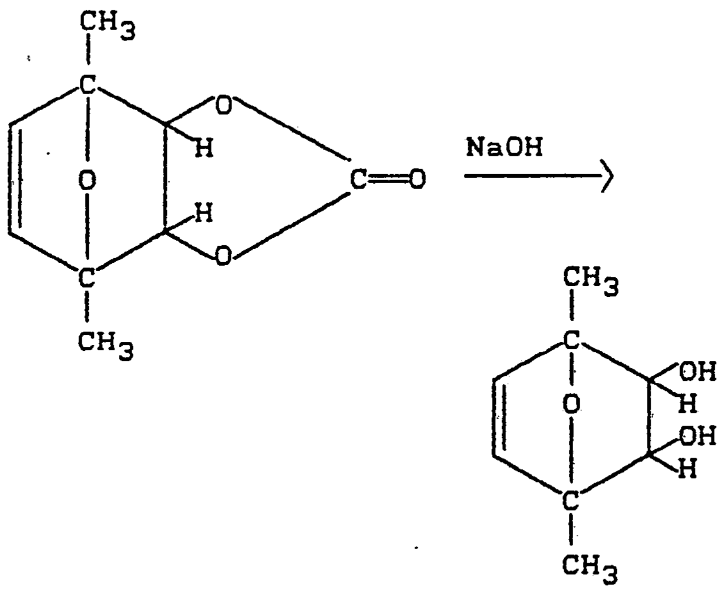


Verwendet man exo-cis-1,4-Dimethyl-2,3-dihydroxy-7-oxabicyclo[2.2.1]hept-5-en und 2-Methyl-benzylchlorid als Ausgangssubstanzen, so kann der Verlauf des erfindungsgemäßen Verfahrens (B) durch das folgende Formelschema wiedergegeben werden:

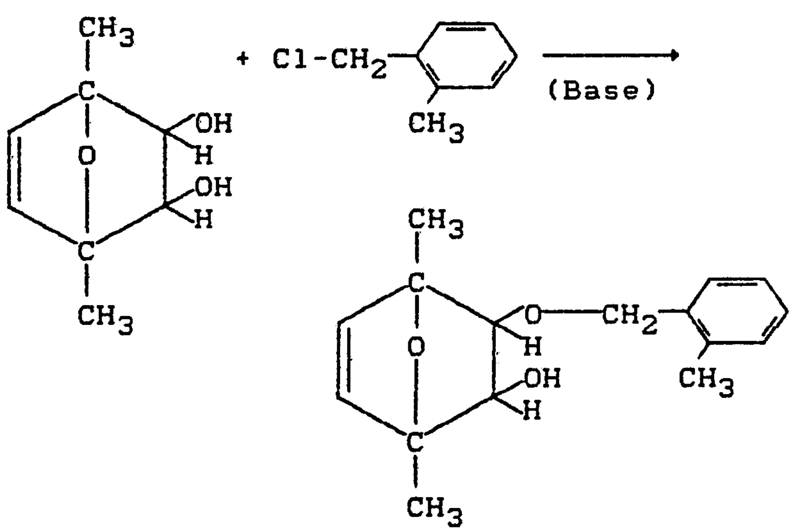

Verwendet man exo-cis-1,4-Dimethyl-2-hydroxy-3-(2-methylbenzyloxy)-7-oxabicyclo[2.2.1]hept-5-en und Methyliodid als Ausgangssubstanzen, so kann der Verlauf des erfindungsgemäßen Verfahrens (C-α) durch das folgende Formelschema wiedergegeben werden:

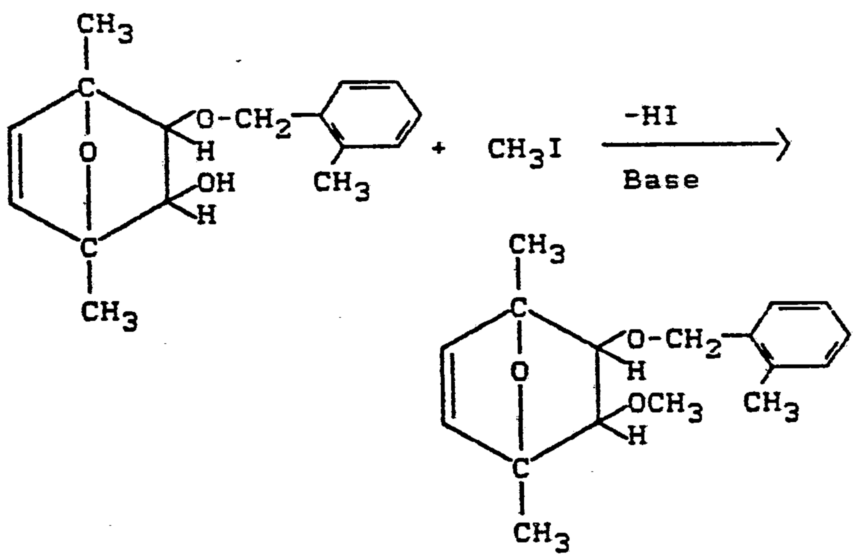


Verwendet man exo-cis-1,4-Dimethyl-2-hydroxy-3-(2-methylbenzyloxy)-7-oxabicyclo[2.2.1]hept-5-en und Acetylchlorid als Ausgangsstoffe, so kann der Verlauf des erfindungsgemäßen Verfahrens (C-β) durch das folgende Formelschema wiedergegeben werden:

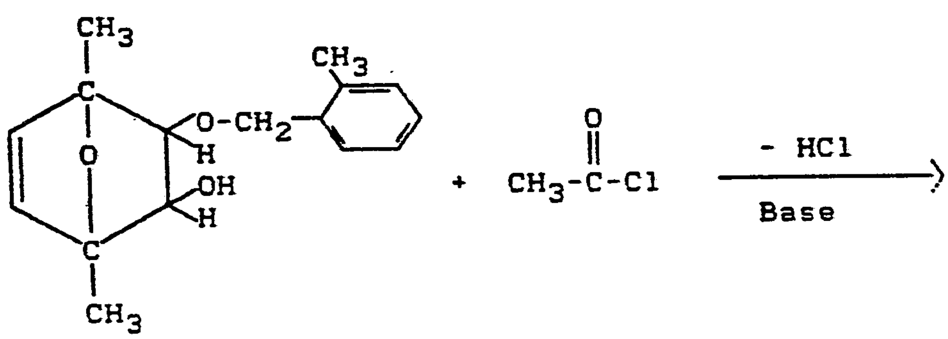

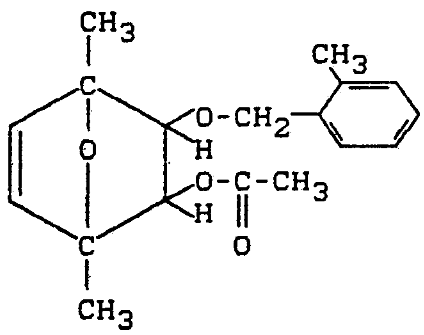

Verwendet man exo-cis-1,4-Dimethyl-2-hydroxy-3-(2-methyl-benzyloxy)-7-oxabicyclo[2.2.1]hept-5-en und Phenylisocyanat als Ausgangsstoffe, so kann der Verlauf des erfindungsgemäßen Verfahrens (C-γ) durch das folgende Formelschema wiedergegeben werden:

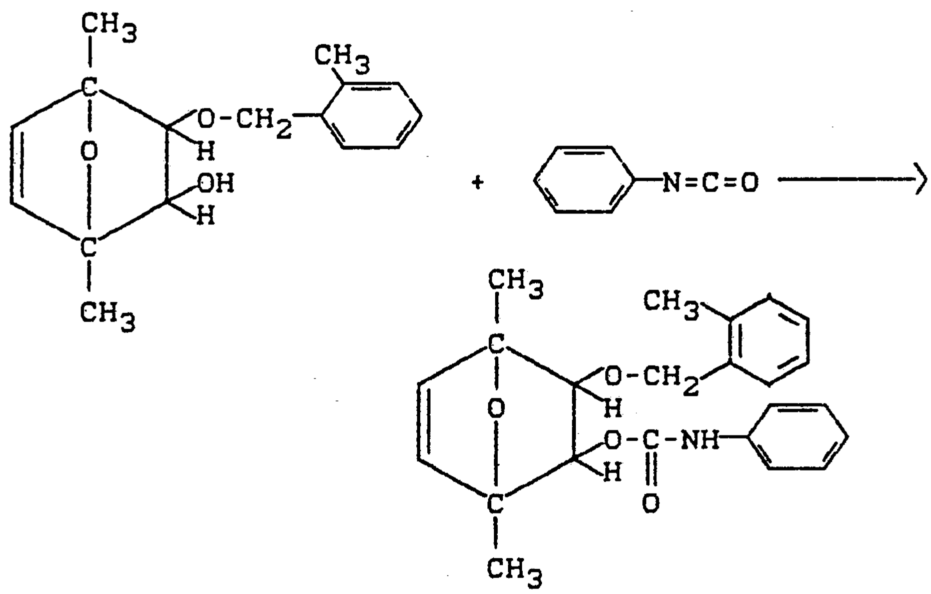

Verwendet man exo-cis-1,4-Dimethyl-2-hydroxy-3-methoxy-7-oxabicyclo[2.2.1]hept-5-en und 2-Chlorbenzylchlorid als Ausgangsstoffe, so kann der Verlauf des erfindungsgemäßen Verfahrens (D) durch das folgende Formelschema wiedergegeben werden:

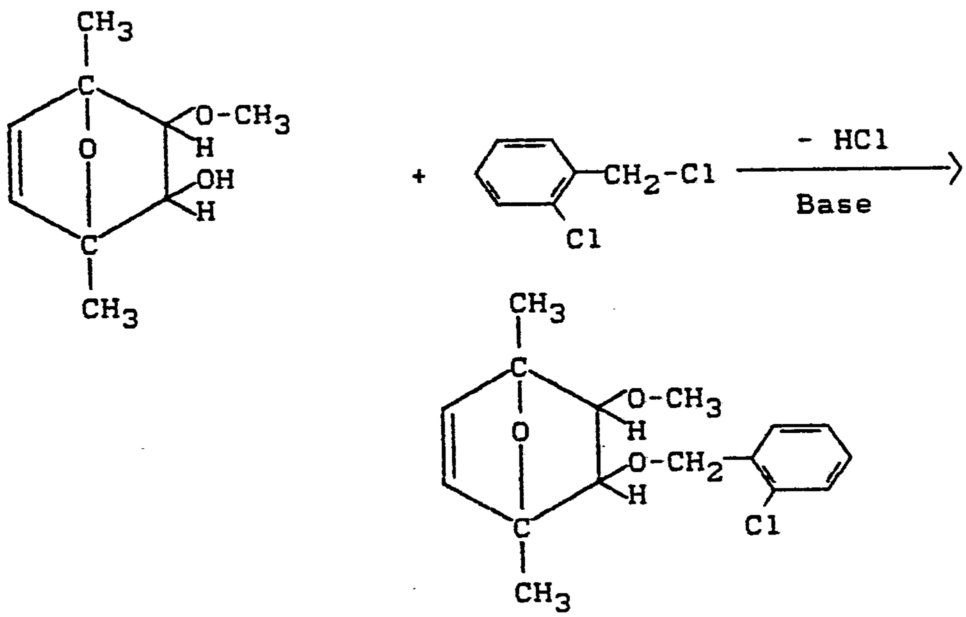


Die als Ausgangsstoffe benötigten Diolcarbonate sind durch die Formel (II) allgemein definiert. In dieser Formel stehen $R^2$ und $R^3$ unabhängig voneinander vorzugsweise für Alkyl mit 1 bis 6 Kohlenstoffatomen, insbesondere für Methyl oder Ethyl.

Die Verbindungen der Formel (II) sind neu und Gegenstand der vorliegenden Erfindung.

Man erhält die Verbindungen der Formel (II), indem man Furane der Formel (VII)

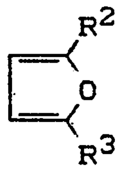

(VII)

in welcher

$R^2$ und $R^3$ die oben angegebene Bedeutung haben,

mit Vinylencarbonat der Formel (VIII)

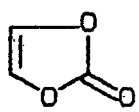

(VIII)

gegebenenfalls in Gegenwart eines Verdünnungsmittels und gegebenenfalls in Gegenwart eines Inertgases umsetzt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und-ethylester, Nitrile wie z. B. Acetonitril und Propionitril, Amide wie z. B. Dimethylformamid, Dimethylacetamid und N-Methylpyrrolidon sowie Dimethylsulfoxid, Tetramethylensulfon und Hexamethylphosphorsäuretriamid.

Als Inertgase kommen dabei Stickstoff sowie praktisch alle Edelgase, insbesondere Argon infrage.

Die Reaktionstemperaturen können bei dem Verfahren zur Herstellung der Derivate der Formel (II) in einem größeren Bereich variiert werden. Im allgemeinen arbeitet man bei Temperaturen zwischen 30° C und 250° C vorzugsweise zwischen 30° C und 200° C.

Zur Durchführung des Verfahrens zur Herstellung der Derivate der Formel (II) setzt man auf 1 Mol der Furane der Formel (VII) im allgemeinen 1 bis 10 Mol, vorzugsweise 3 bis 6 Mol Vinylencarbonat der Formel (VIII) ein.

Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Methoden. Im allgemeinen verfährt man in der Weise, daß man das Reaktionsgemisch destillativ, gegebenenfalls unter vermindertem Druck auftrennt.

Die Furane der Formel (VII) sowie das Vinylencarbonat der Formel (VIII) sind allgemein bekannte Verbindungen der organischen Chemie.

Das erfindungsgemäße Verfahren (A) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Basen kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, infrage, welche im Überschuß eingesetzt werden.

Die Reaktionstemperaturen können bei dem erfindungsgemäßen Verfahren (A) in einem größeren Bereich variiert werden. Im allgemeinen arbeitet man bei Temperaturen zwischen $0^0$ C und $160^0$ C, vorzugsweise zwischen $15^0$ C und $120^0$ C.

Die als Ausgangsstoffe bei dem erfindungsgemäßen Verfahren (B) benötigten Diole der Formel (I-a) sind erfindungsgemäße Verbindungen und erhältlich nach Verfahren (A). In dieser

Formel (I-a) haben $R^2$ und $R^3$ vorzugsweise die bei der Beschreibung der Stoffe der Formel (I) für diese Reste bevorzugt angegebene Bedeutung.

Die bei dem Verfahren (B) weiterhin als Ausgangsstoffe benötigten Halogenide der Formel (III) sind allgemein bekannte Verbindungen der organischen Chemie. In der Formel (III) steht $R^6$ vorzugsweise für gegebenenfalls einfach oder mehrfach, gleich oder verschieden substituiertes Aralkyl mit 6 bis 10 Kohlenstoffatomen im Arylteil und 1 bis 4 Kohlenstoffatomen im Alkylteil, wobei als Arylsubstituenten ausgewählt sind: Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoffatomen und 1 bis 9 gleichen oder verschiedenen Halogenatomen, wie insbesondere Fluor und Chlor.

$R^6$ steht besonders bevorzugt für gegebenenfalls jeweils einfach bis dreifach, gleich oder verschieden durch Wasserstoff, Fluor, Chlor, Methyl, Ethyl, iso-Propyl, n-Propyl, Trifluormethyl, Trifluormethoxy, Trifluormethylthio, Methoxy, Ethoxy, Methylthio und Ethylthiosubstituiertes Benzyl oder Phenethyl.
Als Verdünnungsmittel kommen bei dem erfindungsgemäßen Verfahren (B) praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (B) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kaliummethylat bzw. -ethylat, Hydride wie zum Beispiel Natriumhydrid, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem Verfahren (B) in einem größeren Bereich variiert werden. Im allgemeinen arbeitet man bei Temperaturen zwischen 0° C und 160° C, vorzugsweise zwischen 15° C und 120° C.

Zur Durchführungdes Verfahrens (B) lassen sich Rohprodukte (I-a) auch ohne weitere Reinigung einsetzen.

Zur Durchführung des Verfahrens (B) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt jeweils nach üblichen Methoden.

Die als Ausgangsstoffe bei dem erfindungsgemäßen Verfahren (C) benötigten Oxa-bicyclo[2.2.1]hepten-Derivate sind durch die Formel (I-b) allgemein definiert. In dieser Formel (I-b) haben R, $R^2$ und $R^3$ bevorzugt diejenigen Bedeutungen, die bereits im Zusammenhang mit der Beschreibung der erfindungsgemäßen Stoffe der Formel (I) bevorzugt für diese Reste genannt wurden.

Die Oxa-bicyclo[2.2.1]hepten-Derivate der Formel (I-b) sind erfindungsgemäße Verbindungen und erhältlich nach Verfahren (A) und (B).

Die bei dem erfindungsgemäßen Verfahren (C-α) außerdem als Ausgangsstoffe benötigten Halogenverbindungen sind durch die Formel (IV) allgemein definiert. In dieser Formel steht vorzugsweise

$R^{1-1}$ für Alkyl mit 1 bis 6 Kohlenstoffatomen, Alkenyl mit 3 bis 6 Kohlenstoffatomen, Alkinyl mit 3 bis 6 Kohlenstoffatomen und für gegebenenfalls einfach oder mehrfach, gleich oder verschieden substituiertes Aralkyl mit 6 bis 10 Kohlenstoffatomen im Arylteil und 1 bis 4 Kohlenstoffatomen im Alkylteil, wobei als Arylsubstituenten die bei R vorzugsweise genannten Arylsubstituenten in Frage kommen und

Hal für Chlor und Brom.

Besonders bevorzugt steht $R^{1-1}$ für Methyl, Ethyl, n-Propyl, iso-Propyl, Allyl, Propinyl und gegebenenfalls einfach bis dreifach, gleich oder verschieden durch Fluor, Chlor, Brom, Iod, Methyl und Ethyl substituiertes Benzyl.

Die Halogenverbindungen der Formel (IV) sind allgemein bekannte Verbindungen der organischen Chemie.

Die bei dem erfindungsgemäßen Verfahren (C-β) außerdem als Ausgangsstoffe benötigten Acylhalogenide sind durch die Formel (V) allgemein definiert. In dieser Formel hat $R^4$ vorzugsweise diejenige Bedeutung, die bereits bei der Beschreibung der erfindungsgemäßen Stoffe der Formel (I) für diesen Rest genannt wurde.

Die Acylhalogenide der Formel (V) sind allgemein bekannte Verbindungen der organischen Chemie.

Die bei dem erfindungsgemäßen Verfahren (C-γ) außerdem als Ausgangsstoffe benötigten Isocyanate sind durch die Formel (VI) allgemein definiert. In dieser Formel hat $R^5$ vorzugsweise diejenige Bedeutung, die bereits bei der Beschreibung der erfindungsgemäßen Stoffe der Formel (I) für diesen Rest genannt wurde.

Die Isocyanate der Formel (VI) sind allgemein bekannte Verbindungen der organischen Chemie.

Die erfindungsgemäßen Verfahren (C-α), (C-β) und (C-γ) zur Herstellung der neuen Oxa-bicyclo[2.2.1]hept-en-Derivate der Formel (I) werden vorzugsweise unter Verwendung von Verdünnungsmitteln durchgeführt.

Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutylketon, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säurebindemittel können bei den erfindungsgemäßen Verfahren (C-α), (C-β) und (C-γ) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-, Thalliummethylat bzw. -ethylat, Hydride, wie zum Beispiel Natriumhydrid, ferner aliphatische, aromatische oder hetero cyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (C-α), (C-β) und (C-γ) zur Herstellung der Oxabicyclo[2.2.1]hept-en-Derivate der Formel (I) in einem größeren Bereich variiert werden. Im allgemeinen arbeitet man bei Temperaturen zwischen 0° C und 160° C, vorzugsweise zwischen 15° C und 120° C.

Die erfindungsgemäßen Verfahren (C-α), (C-β) und (C-γ) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (C-α), (C-β) und (C-γ) werden die jeweils benötigten Ausgangsstoffe im allgemeinen in angenähert äquimolaren Mengen eingesetzt.

Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die bei dem erfindungsgemäßen Verfahren (D) als Ausgangsstoffe benötigten Ether-Derivate sind durch die Formel (I-c) allgemein definiert. In dieser Formel haben $R^1$, $R^2$ und $R^3$ bevorzugt diejenige Bedeutung, die bereits im Zusammenhang mit der Beschreibung der erfindungsgemäßen Stoffe der Formel (I) bevorzugt für diese Reste genannt wurde.

Die Ether-Derivate der Formel (I-c) sind erfindungsgemäße Verbindungen und erhältlich nach Verfahren (B) und (C).

Die bei dem erfindungsgemäßen Verfahren (D) außerdem als Ausgangsstoffe benötigten Halogenverbindungen der Formel (III) sind allgemein bekannte Verbindungen der organischen Chemie.

Das erfindungsgemäße Verfahren (D) zur Herstellung der neuen Oxa-bicyclo[2.2.1]hepten-Derivate der Formel (I) wird vorzugsweise unter Verwendung von Verdünnungsmitteln durchgeführt. Als Verdünnungsmittel kommen dabei praktisch alle inerten organischen Lösungsmittel in Frage, welche bereits bei der Beschreibung von Verfahren (B) genannt wurden.

Als Säurebindemittel können bei dem erfindungsgemäßen Verfahren (D) alle üblicherweise für derartige Umsetzungen verwendbaren Säurebindemittel eingesetzt werden. Vorzugsweise in Frage kommen die Säurebindemittel, die bereits bei der Beschreibung von Verfahren (B) genannt wurden.

Die Reaktionstemperaturen können bei dem erfindungsgemäßen Verfahren (D) zur Herstellung der Oxa-bicyclo[2.2.1]hepten-Derivate der Formel (I) in einem größeren Bereich variiert werden. Im allgemeinen arbeitet man bei Temperaturen zwischen 0° C und 160° C vorzugsweise zwischen 15° C und 120° C.

Das erfindungsgemäße Verfahren (D) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (D) werden die jeweils benötigten Ausgangsstoffe im allgemeinen in angenähert äquimolaren Mengen eingesetzt, Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in mono- und dikotylen Kulturen im Vorauflaufverfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit,

Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier-und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen können bekannte Herbizide verwendet werden wie z.B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 2-Chlor-N-{[4-methoxy-6-methyl-1,3,5-triazin-2-yl)-amino]-carbonyl}-benzolsulfonamid, Ethyl-2-{[(4-Chlor-6-methoxy-2-pyrimidinyl)-aminocarbonyl]aminosulfonyl}-benzoat, 2-Ethylamino-6-(1,1-dimethylethyl-amino)-4-methylthio-1,3,5-triazin, 4-Amino-6-(1,1-dimethylethyl)-3-methylthio-1,2,4-triazin-5(4H)-on, 4-Amino-6-(1,1-dimethylethyl)-3-ethylthio-1,2,4-triazin-5(4H)-on, 1-Amino-6-ethylthio-3- (2,2-dimethylpropyl)-1,3,5-triazin-2,4-(1H,3H)-dion, 5-(2-Chlor-4-trifluormethyl-phenoxy)-N-methylsulfonyl-2-nitrobenzamid, (2-Ethoxy-1-methyl-2-oxo-ethyl)-5-(2-chlor-4-trifluormethyl-phenoxy)-2-nitrobenzoat, 2-[4-(2,4-Dichlorphenoxy)-phenoxy]-propionsäuremethylester, 2-{4-[(3-Chlor-5-trifluormethyl-2-pyridinyl)oxy]-phenoxy}-propionsäure, das R-Enantiomere des 2-{4-[(3,5-Dichlor-2-pyridinyl)oxy]-phenoxy}-propionsäure-(trimethylsilyl)-methylesters, 2,4-Dichlorphenoxyessigsäure, 2-(2,4-Dichlorphenoxy)-propionsäure, 4-Chlor-2-methyl-phenoxy-essigsäure, 2-(4-Chlor-2-methylphenoxy)-propion säure, 3,5-Diiod-4-hydroxy-benzonitril,-3,5-Dibrom-4-hydroxy-benzonitril, 2-[5-Methyl-5-(1-methylethyl)-4-oxo-2-imidazolin-2-yl]-3-pyridincarbon-

säure, 2-(1-Ethoxyamino-butyliden)-5-(2-ethylthiopropyl)-1,3-cyclohexandion, [(4-Amino-3,5-dichlor-6-fluor-2-pyridinyl)oxy]-essigsäure, 3-Isopropyl-2,1,3-benzothiadiazinon-(4)-2,2-dioxid, N-Methyl-2-(1,3-benzthiazol-2-yloxy)-acetanilid, α-Chlor-2',6'-diethyl-N-(2-propoxyethyl)-acetanilid, Propionsäure-3,4-dichloranilid, Hexahydro-1H-azepin-1-carbamidsäure-thiolethylester, 2-{[4-(2,4-Dichlorbenzoxyl)-1,3-dimethyl-1H-pyrazol-5-yl]-oxy}-1-phenylethanon. Einige Mischungen besitzen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

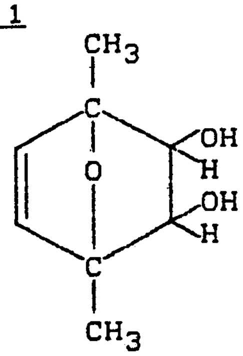

(I-1)

(Verfahren A)

30 g (0,153 Mol) exo-cis-1,4-Dimethyl-7-oxabicyclo[2.2.1]-hept-5-en-2,3-diolcarbonat werden in 500ml 5%iger Natronlauge 1,5 Stunden bei Raumtemperatur gerührt. Die Lösung wird anschließend mit Natriumchlorid gesättigt und 3 - 4 mal mit Tetrahydrofuran extrahiert. Der pH-Wert wird auf 6,5 eingestellt. Anschließend wird einmal mit Essigester extrahiert. Die vereinigten organischen Phasen werden getrocknet und eingeengt. Das Rohprodukt wird für die weitere Umsetzung ohne weitere Reinigung eingesetzt.
Man erhält 22 g (92,4% der Theorie) exo-cis-1,4-Dimethyl-2,3-dihydroxy-7-oxabicyclo[2.2.1]hept-5-en.

Herstellung des Ausgangsproduktes

Beispiel II-1:

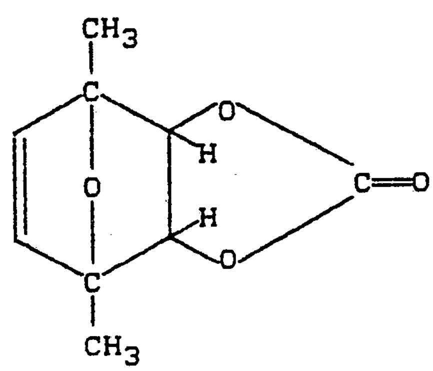

(II-1)

441 g (5,18 Mol) Vinylencarbonat und 70 g (1,022 Mol) 2,5-Dimethylfuran werden unter Argon 24 Stunden unter Rückfluß und Magnetrührung erhitzt. Anschließend wird das überschüssige Vinylencarbonat über eine 30 cm-Vigreux-Kolonne im Wasserstrahlvakuum bei ca. 50° C abdestilliert.

Man erhält 120 g (66% der Theorie) eines 60:40 Gemisches der beiden exo/endo-Cycloadditionsprodukte, die im Hochvakuum destillativ getrennt werden können. Die in der exo-Form vorliegende Substanz destilliert bei Kp 160° C/1 mbar.

Ausbeute: 30 g exo-cis-1,4-Dimethyl-7-oxabicyclo[2.2.1]hept-5-en-2,3-diolcarbonat.

<u>Beispiel 2</u>

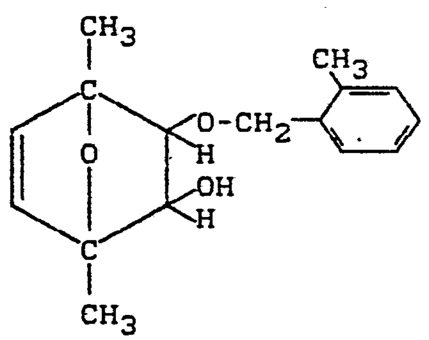

(I-2)

(Verfahren B)

4 g (0,026 Mol) exo-cis-1,4-Dimethyl-2,3-dihydroxy-7-oxabicyclo[2.2.1]hept-5-en werden mit äquimolaren Mengen Kaliumcarbonat (3,5 g) und 2-Methyl-benzylchlorid (3,6 g) in 50 ml Toluol eine Stunde bei Raumtemperatur und anschließend 12 Stunden unter Rückfluß gerührt. Das abgekühlte Reaktionsgemisch wird filtriert und eingeengt und das Rohprodukt im Hochvakuum destilliert.

Man erhält 5,8 g (86,5% der Theorie) exo-cis-1,4-Dimethyl-2-hydroxy-3-(2-methylbenzyloxy)-7-oxabicyclo[2.2.1]hept-5-en vom Siedepunkt 110°C/0,1 mbar, mit einem Brechungsindex $n_D^{20}$ =1,5168.

Beispiel 3

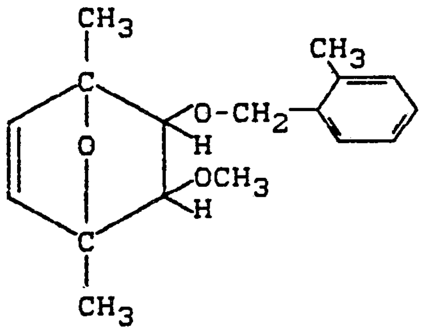

(I-3)

(Verfahren (C-α))
1,5g (0,0057 Mol) exo-cis-1,4-Dimethyl-2-hydroxy-3-(2-methyl-benzyloxy)-7-oxabicyclo[2.2.1]hept-5-en werden in 20 ml Dimethylformamid gelöst und nacheinander bei Raumtemperatur mit 0,165g (0,0057 Mol) 80%igem Natriumhydrid und 0,813 g (0,0057 Mol) Methyliodid versetzt. Anschließend wird eine Stunde bei Raumtemperatur und dann 12 Stunden bei 70°C gerührt. Das abgekühlte Reaktionsgemisch wird in 50 ml Wasser gegossen, mit Methylenchlorid extrahiert, getrocknet, eingeengt und im Hochvakuum destilliert.
Man erhält 1 g (63 % der Theorie) exo-cis-1,4-Dimethyl-2-methoxy-3-(2-methyl-benzyloxy)-7-oxabicyclo[2.2.1]hept-5-en vom Brechungsindex $n_D^{20}$ = 1,5172.

Beispiel 4

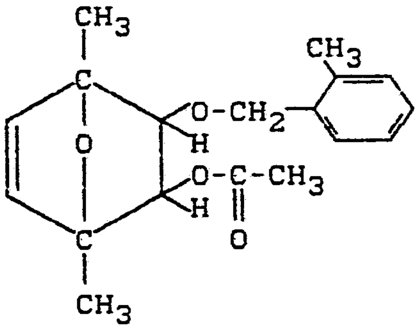

(I-4)

(Verfahren (C-β))

5 g (0,0192 Mol) exo-cis-1,4-Dimethyl-2-hydroxy-3-(2-methyl-benzyloxy)-7-oxabicyclo[2.2.1]hept-5-en werden in 50 ml Dimethylformamid gelöst, mit 0,55 g (0,0192 Mol) 80%igem Natriumhydrid versetzt und anschließend mit 1,54 g (0,0192 Mol) Acetylchlorid 1 Stunde bei Raumtemperatur und weitere 12 Stunden bei 70°C gerührt. Nach dem Abkühlen wird die Reaktionsmischung auf 50 ml Wasser gegossen und mit Methylenchlorid extrahiert. Die organische Phase wird getrocknet, eingeengt und der Rückstand im Hochvakuum destilliert.
Man erhält 1,2 g (21% der Theorie) exo-cis-1,4-Dimethyl-2-acetoxy-3-(2-methylbenzyloxy)-7-oxabicyclo[2.2.1]hept-5-en vom Brechungsindex $n_D^{20}$ =1,5138.

Beispiel 5

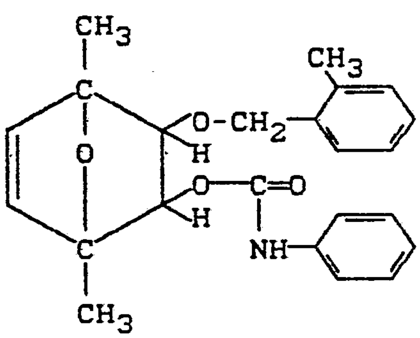

(I-5)

(Verfahren C-γ)

5 g (0,0192 Mol) exo-cis-1,4-Dimethyl-2-hydroxy-3-(2-methyl-benzyloxy)-7-oxabicyclo[2.2.1]hept-5-en werden in 50 ml Toluol gelöst. Danach werden katalytische Mengen Triethylamin und Desmorapid Z mit 3,5 g (0,027 Mol) Phenylisocyanat zugegeben und 12 Stunden unter Rückfluß gerührt. Dann werden 50 ml Wasser zugegeben und mit Essigester extrahiert. Die organische Phase wird getrocknet und eingeengt. Der kristalline Rückstand wird mit Ether digeriert, der unlösliche Diphenylharnstoff abfiltriert und das Filtrat eingeengt.

Man erhält 6,5 g (90% der Theorie) exo-cis-1,4-Dimethyl-2-phenylcarbamoyl-3-(2-methyl-benzyloxy)-7-oxabicyclo[2.2.1]-hept-5-en mit Fp. 125°C.

Beispiel 6

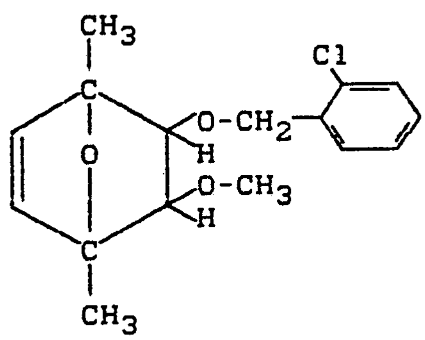

(I-6)

(Verfahren D)

4 g (0,023 Mol) exo-cis-1,4-Dimethyl-2-hydroxy-3-methoxy-7-oxabicyclo[2.2.1]hept-5-en werden in 50 ml Dimethylformamid gelöst und anschließend mit 0,66 g (0,023 Mol) 80%igem Natriumhydrid versetzt. Nach 15 Minuten gibt man bei Raumtemperatur 3,8 g (0,023 Mol) 2-Chlorbenzylchlorid zu und rührt 12 Stunden bei 50°C. Die Aufarbeitung erfolgt, wie bereits bei Verfahren (C-α) beschrieben.

Man erhält 5,7 g (84% der Theorie) exo-cis-1,4-Dimethyl-2-methoxy-3-(2-chlorbenzyloxy)-7-oxabicyclo[2.2.1]hept-5-en vom Brechungsindex $n_D^{20}$ = 1,5273.

Beispiel 7

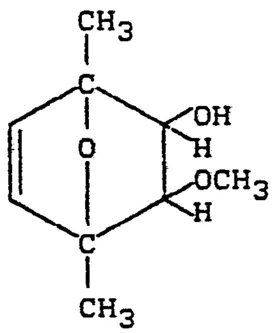

(I-7)

(Verfahren C-α)

5 g (0,032 Mol) exo-cis-1,4-Dimethyl-2,3-dihydroxy-7-oxabicyclo[2.2.1]hept-5-en werden in 50 ml Acetonitril gelöst und bei Raumtemperatur mit 7,99 g (0,032 Mol) Thalliumethylat unter Rühren versetzt, wobei sich nach ca. 30 Minuten ein weißer Niederschlag bildet. Dann werden 4,96 g (0,035 Mol) Methyliodid zugetropft und bei 50° C 12 Stunden gerührt. Der orangefarbene Niederschlag wird abgesaugt, mit Acetonitril gewaschen, das Filtrat wird eingeengt und im Hochvakuum destilliert.
Man erhält 4 g (73,5% der Theorie) exo-cis-1,4-Dimethyl-2-methoxy-3-hydroxy-7-oxabicyclo[2.2.1]hept-5-en vom Siedepunkt Kp: 95° C/0,1 mbar.

Analog den Herstellungsbeispielen (I-1) bis (I-7) und entsprechend den angegebenen Verfahren können die folgenden Endprodukte der Formel (I) erhalten werden:

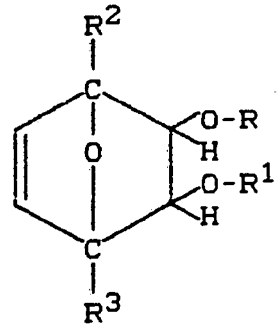

(I)

Tabelle 2

| Bsp. Nr. | $R^2$ | $R^3$ | $R^1$ | R | Brechungsindex ($n_D^{20}$) bzw. Siedepunkt (°C) |
|---|---|---|---|---|---|
| I-8 | $CH_3$ | $CH_3$ | $C_2H_5$ | $-CH_2-$ 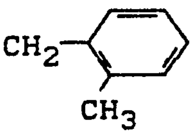 | 1,5139 |
| I-9 | $CH_3$ | $CH_3$ | $n-C_3H_7$ | $-CH_2-$ 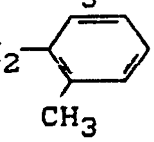 | 1,5046 |
| I-10 | $CH_3$ | $CH_3$ | $CH_3$ | $-CH_2-$ 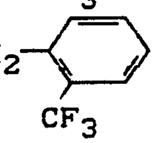 | 1,4842 |
| I-11 | $CH_3$ | $CH_3$ | $CH_3$ | $-CH_2-$ 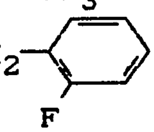 | 1,5098 |
| I-12 | $CH_3$ | $CH_3$ | $-CO-$ 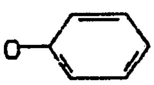 | $-CH_2-$ 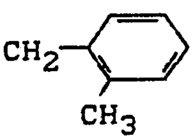 | 1,5372 |
| I-13 | $CH_3$ | $CH_3$ | $-CO-NH-CH_3$ | $-CH_2-$ 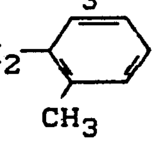 | 1,5160 |
| I-14 | $CH_3$ | $CH_3$ | $CH_3$ | $-CH_2-$ 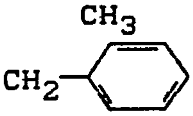 | 1,5120 |

Tabelle 2 (Fortsetzung)

| Bsp. Nr. | $R^2$ | $R^3$ | $R^1$ | R | Brechungsindex ($n_D^{20}$) bzw. Siedepunkt (°C) |
|---|---|---|---|---|---|
| I-15 | $CH_3$ | $CH_3$ | $-CH_2$ 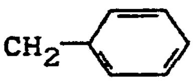 | $-CH_2$ 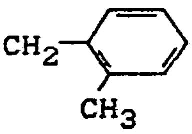 | 130°C/0,1 mbar |
| I-16 | $CH_3$ | $CH_3$ | H | $-CH_2$ 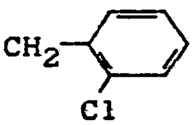 | 73°C |
| I-17 | $CH_3$ | $CH_3$ | H | $-CH_2$ 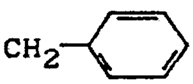 | 125°C/0,1 mbar |
| I-18 | $CH_3$ | $CH_3$ | H | $-CH_2$ 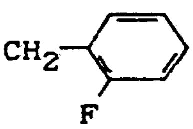 | 1.5329 |
| I-19 | $CH_3$ | $CH_3$ | H | $-CH_2$ 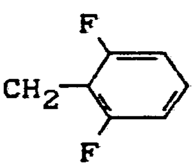 | 1.4974 |
| I-20 | $CH_3$ | $CH_3$ | $-CH_2-C{\equiv}CH$ | $-CH_2$ 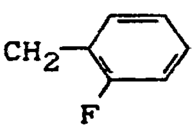 | 1.5158 |
| I-21 | $CH_3$ | $CH_3$ | $CH_3$ | $-CH_2$ 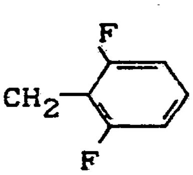 | 1.5011 |

Anwendungsbeispiele:

In den folgenden Anwendungsbeispielen wurde die nachstehend aufgeführte Verbindung als Vergleichssubstanz eingesetzt:

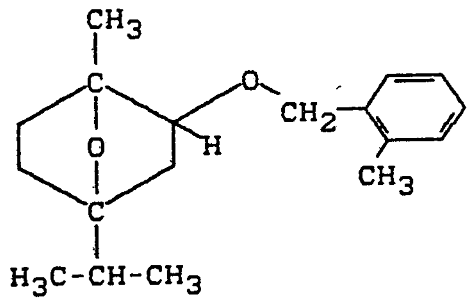

(A)

exo-1-Methyl-4-iso-propyl-2-(2-methylbenzyloxy)-7-oxabicyclo[2.2.1]heptan
(bekannt aus EP-A-81 893).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt z.B. die Verbindung gemäß Herstellungsbeispiel (I-11) neben einer allgemeinen guten herbiziden Wirkung gegen Unkräuter wie beispielsweise Agropyron, Alopecurus und Echinochloa eine deutlich bessere Nutzpflanzenverträglichkeit, insbesondere in Reis, als die Vergleichssubstanz (A).

## Patentansprüche

1. Oxa-bicyclo[2.2.1]hepten-Derivate der Formel (I)

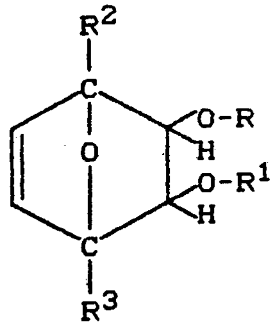

(I)

in welcher

R für Wasserstoff oder gegebenenfalls substituiertes Aralkyl steht,

$R^1$ für Wasserstoff, Alkyl, Alkenyl, Alkinyl gegebenenfalls substituiertes Aralkyl oder für den Rest $-CO-R^4$ steht, worin

$R^4$ für Alkyl oder gegebenenfalls substituiertes Phenyl oder für den Rest $-NH-R^5$ steht, worin

$R^5$ für Alkyl oder gegebenenfalls substituiertes Phenyl steht und

$R^2$ und $R^3$ gleich oder verschieden sind und für Alkyl stehen.

2. Oxa-bicyclo[2.2.1]hepten-Derivate der Formel (I) gemäß Anspruch 1, in welcher

R für Wasserstoff oder gegebenenfalls einfach oder mehrfach, gleich oder verschieden substituiertes Aralkyl mit 6 bis 10 Kohlenstoffatomen im Arylteil und 1 bis 4 Kohlenstoffatomen im Alkylteil, wobei als Arylsubstituenten ausgewählt sind: Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoffatomen und 1 bis 9 gleichen oder verschiedenen Halogenatomen,

$R^1$ für Wasserstoff, Alkyl mit 1 bis 6 Kohlenstoffatomen, Alkenyl mit 3 bis 6 Kohlenstoffatomen, Alkinyl mit 3 bis 6 Kohlenstoffatomen, gegebenenfalls einfach oder mehrfach, gleich oder verschieden substituiertes Aralkyl mit 6 bis 10 Kohlenstoffatomen im Arylteil und 1 bis 4 Kohlenstoffatomen im Alkylteil steht, wobei als Arylsubstituenten die bei R genannten Arylsubstituenten infrage kommen; ferner für den Rest $-COR^4$ steht, wobei

$R^4$ für Alkyl mit 1 bis 4 Kohlenstoffatomen oder gegebenenfalls einfach oder mehrfach, gleich oder verschieden substituiertes Phenyl steht, wobei als Phenylsubstituenten die bei R genannten Arylsubstituenten infrage kommen, oder für den Rest $-NHR^5$ steht, wobei

$R^5$ für Alkyl mit 1 bis 4 Kohlenstoffatomen oder für gegebenenfalls einfach oder mehrfach, gleich oder verschieden substituiertes Phenyl steht, wobei als Phenylsubstituenten die bei R genannten Aryl substituenten infrage kommen, und

$R^2$ und $R^3$ gleich oder verschieden sind und für Alkyl mit 1 bis 6 Kohlenstoffatomen stehen.

3. Oxa-bicyclo[2,2,1]hepten-Derivate der Formel (I) gemäß Anspruch 1, die in der exo-Form vorliegen und in denen

R für Wasserstoff oder für jeweils einfach bis dreifach, gleich oder verschieden durch Wasserstoff, Fluor, Chlor, Methyl, Ethyl, iso-Propyl, n-Propyl, Trifluormethyl, Trifluormethoxy, Trifluormethylthio, Methoxy, Ethoxy, Methylthio und Ethylthio substituiertes Benzyl oder Phenethyl steht,

und

$R^1$ für Wasserstoff, Methyl, Ethyl, n-Propyl, iso-Propyl, Allyl oder Propinyl steht; ferner für Benzyl steht, welches gegebenenfalls einfach bis dreifach, gleich oder verschieden durch Fluor, Chlor, Brom, Iod, Methyl und Ethyl substituiert ist; sowie für den Rest $-COR^4$ steht, wobei

$R^4$ für Methyl, Ethyl, Phenyl oder den Rest $-NHR^5$ steht, wobei

$R^5$ für Methyl, Ethyl oder Phenyl steht und

$R^2$ und $R^3$ gleich oder verschieden sind und für Methyl oder Ethyl stehen.

4. Verfahren zur Herstellung von Oxa-bicyclo[2.2.1]-hepten-Derivaten der Formel (I)

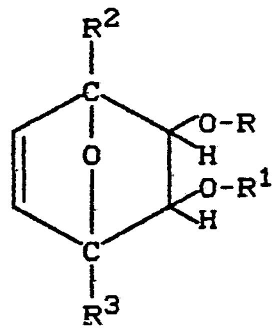

(I)

in welcher

R für Wasserstoff oder gegebenenfalls substituiertes Aralkyl steht,

$R^1$ für Wasserstoff, Alkyl, Alkenyl, Alkinyl gegebenenfalls substituiertes Aralkyl oder für den Rest $-CO-R^4$ steht,
worin

$R^4$ für Alkyl oder gegebenenfalls substituiertes Phenyl oder für den Rest $-NH-R^5$ steht,

worin

$R^5$ für Alkyl oder gegebenenfalls substituiertes Phenyl steht und

$R^2$ und $R^3$ gleich oder verschieden sind und für Alkyl stehen,

dadurch gekennzeichnet, daß man

A) Diolcarbonate der Formel (II)

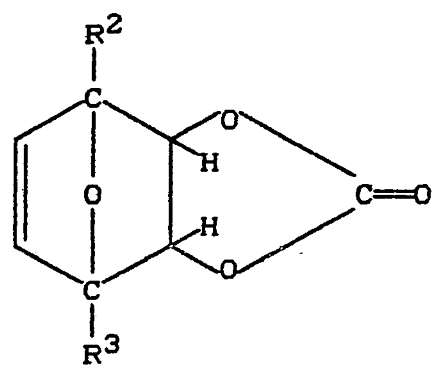

(II)

in welcher

$R^2$ und $R^3$ gleich oder verschieden sind und für Alkyl stehen,

gegebenenfalls in Gegenwart eines Verdünnungsmittels und in Gegenwart einer Base hydrolisiert,

oder daß man

B) die nach Verfahren (A) erhältlichen Diole der Formel (I-a)

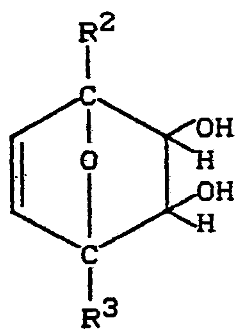

(I-a)

in welcher

$R^2$ und $R^3$ die oben angegebene Bedeutung haben,

mit Halogeniden der Formel (III)

$$R^6 - Hal \quad (III)$$

in welcher

$R^6$ für gegebenenfalls substituiertes Aralkyl steht und

Hal für Halogen steht,

gegebenenfalls in Gegenwart eines Verdünnungsmittels und gegebenenfalls in Gegenwart eines Säurebindemittels umsetzt,

oder daß man

C) Oxa-bicyclo[2.2.1]hepten-Derivate der Formel (I-b)

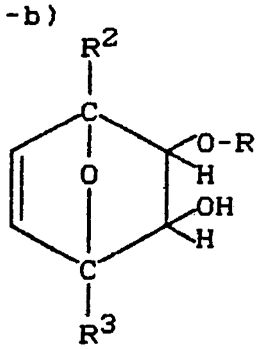

(I-b)

in welcher

$R^2$, $R^3$ und R die oben angegebene Bedeutung haben,

α) mit Halogeniden der Formel (IV)

$R^{1-1}$-Hal(IV)

in welcher

$R^{1-1}$ für Alkyl, Alkenyl, Alkinyl oder gegebenenfalls substituiertes Aralkyl steht und

Hal für Halogen steht,

gegebenenfalls in Gegenwart eines Verdünnungsmittels und gegebenenfalls in Gegenwart einer Base umsetzt,
oder

β) mit Acylhalogeniden der Formel (V)

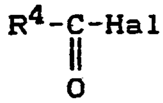 (V)

in welcher

$R^4$ die oben angegebene Bedeutung hat und

Hal für Halogen steht,

gegebenenfalls in Gegenwart eines Säurebindemittels sowie gegebenenfalls in Gegenwart eines Verdünnungsmittels umsetzt,

oder

γ) mit Isocyanaten der Formel (VI)

$R^5-N=C=O$ (VI)

in welcher

$R^5$ die oben angegebene Bedeutung hat,

gegebenenfalls in Gegenwart eines Verdünnungsmittels umsetzt;

oder daß man

D) Ether-Derivate der Formel (I-c)

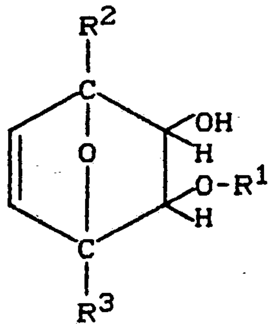

(I-c)

in welcher

$R^1$, $R^2$ und $R^3$ die oben angegebene Bedeutung haben,

mit Verbindungen der Formel (III)

$R^6$-Hal (III)

in welcher

$R^6$ die oben angegebene Bedeutung hat und

Hal für Halogen steht,

gegebenenfalls in Gegenwart einer Base und gegebenenfalls in Gegenwart eines Verdünnungsmittels umsetzt.

5. Herbizide Mittel, gekennzeichnet durch einen Gehalt an mindestens einem Oxa-bicyclo[2.2.1]hepten-Derivat der Formel (I) gemäß den Anspruchen 1 bis 4.

6. Verfahren zur Bekämpfung von Unkräutern, dadurch gekennzeichnet, daß man Oxa-bicyclo[2.2.1]hepten-Derivate der Formel (I) gemäß den Ansprüchen 1 bis 4 auf die Unkräuter und/oder ihren Lebensraum einwirken läßt.

7. Verwendung von Oxa-bicyclo[2.2.1]hepten-Derivate der Formel (I) gemäß den Ansprüchen 1 bis 4 zur Bekämpfung von Unkräutern.

8. Verfahren zur Herstellung von herbiziden Mitteln, dadurch gekennzeichnet, daß man Oxa-bicyclo[2.2.1]-hepten-Derivate der Formel (I) gemäß den Ansprüchen 1 bis 4 mit Streckmitteln und/oder oberflächenaktiven Substanzen vermischt.

9. Diolcarbonate der Formel (II)

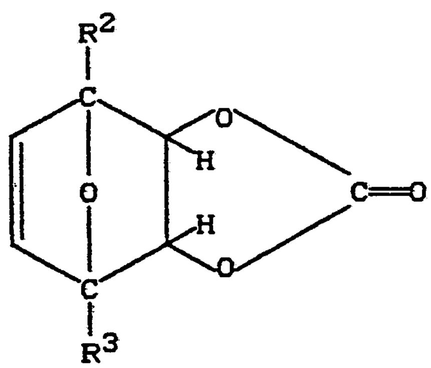

(II)

in welcher

$R^2$ und $R^3$ gleich oder verschieden sind und für Alkyl stehen.

10. Verfahren zur Herstellung von Diolcarbonaten der Formel (II)

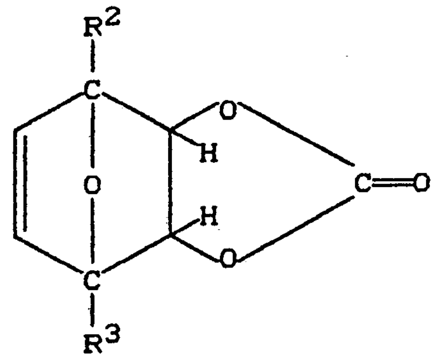

(II)

in welcher

$R^2$ und $R^3$ gleich oder verschieden sind und für Alkyl stehen,

dadurch gekennzeichnet, daß man Furane der Formel (VII)

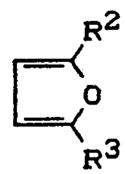

(VII)

in welcher

$R^2$ und $R^3$ die oben angegebene Bedeutung haben,

mit Vinylencarbonat der Formel (VIII)

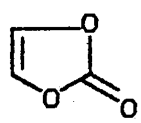

(VIII)

gegebenenfalls in Gegenwart eines Verdünnungsmittels und gegebenenfalls in Gegenwart eines Inertgases umsetzt.

Europäisches Patentamt

**0 247 486**

## EUROPÄISCHER RECHERCHENBERICHT

EP 87 10 7166

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | JOURNAL OF THE AMERICAN CHEMICAL SOCIETY, Band 77, 1955, Seiten 3789-3793; M.S. NEWMAN et al.: "Synthesis and Reactions of Vinylene Carbonate" * Seite 3792, rechte Spalte, Seite 3793, linke Spalte, Abschnitt "e. Furan" * | 1,4,9,10 | C 07 D 493/08<br>C 07 D 493/18<br>A 01 N 43/90 //<br>(C 07 D 493/08<br>C 07 D 307:00<br>C 07 D 307:00 )<br>(C 07 D 493/18<br>C 07 D 317:00<br>C 07 D 307:00<br>C 07 D 307:00 ) |
| A | JOURNAL OF THE AMERICAN CHEMICAL SOCIETY, Band 95, Nr. 21, 1973, Seiten 7161-7162; W.K. ANDERSON et al.: "2-Phenyl- and 2-Thiono-1,3-dioxol-4-ene. Alternatives to Acetylene as Dienophiles in the Diels-Alder Reaction" * Seite 7161, rechte Spalte, Zeilen 1-4, Schema II * | 1,4,9,10 | |
| | --- -/- | | |

RECHERCHIERTE SACHGEBIETE (Int. Cl.4)

A 01 N 43/90
C 07 D 493/00

Der vorliegende Recherchenbericht wurde für alle Patentansprüche erstellt.

| Recherchenort | Abschlußdatum der Recherche | Prüfer |
|---|---|---|
| BERLIN | 20-08-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

0 247 486

EP 87 10 7166

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 53, Nr. 3, 10. Februar 1959, Spalten 2273f-2274a, Columbus, Ohio, US; G. JACOB et al.: "Stereochemistry of ascaridole glycol"; & BULL. SOC. CHIM., FRANCE, 1958, 734-736 in Verbindung mit CHEMICAL ABSTRACTS, Band 53, Subject Index A-I, 1959, Columbus, Ohio, US; * Seite 1032s, rechte Spalte, Stichwort 4,7-Epoxy-1,3-benzodioxol-2-one, hexahydro-4-isopropyl-7-methyl- * --- | 1,9 | |
| D,A | EP-A-0 081 893 (SHELL INTERNATIONALE RESEARCH MAATSCHAPPIJ B.V.) * Ansprüche 1, 23, 24; Beispiele 15, 16, 20 * --- | 1,5-8 | RECHERCHIERTE SACHGEBIETE (Int. Cl.4) |
| A | CHEMICAL ABSTRACTS, Band 77, Nr. 21, 20. November 1972, Seite 424, Zusammenfassungsnr. 139803x, Columbus, Ohio US; & JP - A - 72 27511 (T. SHONO) 22.07.1972 --- | 1 | |
| A | US-A-4 515 972 (J. DAS et al.) * Anspruch 17 * ----- | 9 | |

Der vorliegende Recherchenbericht wurde für alle Patentansprüche erstellt.

| Recherchenort | Abschlußdatum der Recherche | Prüfer |
|---|---|---|
| BERLIN | 20-08-1987 | HASS C V F |

KATEGORIE DER GENANNTEN DOKUMENTE
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82